(19) Europäisches Patentamt
European Patent Office
Office européen des brevets

(11) Publication number: **0 502 195 A1**

(12) **EUROPEAN PATENT APPLICATION published in accordance with Art. 158(3) EPC**

(21) Application number: **91915146.4**

(22) Date of filing: **29.08.91**

(86) International application number: **PCT/JP91/01150**

(87) International publication number: **WO 92/05268 (02.04.92 92/08)**

(51) Int. Cl.[5]: **C12P 13/06**, //(C12P13/06, C12R1:07),(C12P13/06, C12R1:55),(C12P13/06, C12R1:465),(C12P13/06, C12R1:20),(C12P13/06, C12R1:13),(C12P13/06, C12R1:265),(C12P13/06, C12R1:445),(C12P13/06, C12R1:01)

(30) Priority: **20.09.90 JP 248923/90**

(43) Date of publication of application: **09.09.92 Bulletin 92/37**

(84) Designated Contracting States: **DE FR GB IT NL**

(71) Applicant: **NIPPON STEEL CORPORATION**
**6-3 Otemachi 2-chome Chiyoda-ku**
**Tokyo 100-71(JP)**

(72) Inventor: **KATO, Yasuo, Nippon Steel Corp., Adv. Materials**
**& Tech. Res. Lab., 1618, Ida, Nakahara-ku**
**Kawasaki-chi, Kanagawa 211(JP)**
Inventor: **FUKUMOTO, Kenji, Nippon Steel Corp., Adv. Material**
**& Tech. Res. Lab., 1618, Ida, Nakahara-ku**
**Kawasaki-shi, Kanagawa 211(JP)**

(74) Representative: **Geering, Keith Edwin et al**
**REDDIE & GROSE 16 Theobalds Road**
**London WC1X 8PL(GB)**

(54) **PROCESS FOR PRODUCING L--g(b)-HALOALANINE.**

(57) A process for producing L-$\beta$-chloroalanine or L-$\beta$-bromoalanine, which comprises bringing 3-chloro-2-ketobutyric acid or 3-bromo-2-ketobutyric acid as a substrate into contact with an active substance capable of asymmetrically reducing and aminating 2-ketobutyric acid in the presence of the reduced form nicotinamide-adenine-dinucleotide (NADH) and an ammonia source. Examples of the active substance include those having alanine dehydrogenase, leucine dehydrogenase and phenylalanine dehydrogenase activities. If necessary, the above process may incorporate an NADH regenerating system.

EP 0 502 195 A1

## TECHNICAL FIELD

The present invention relates to a process for producing L-β-haloalanine using an enzymatic conversion, and specifically, to a process for enzymatically producing a corresponding L-β-haloalanine from a substrate, 3-chloro- or 3-bromo-2-ketobutyric acid.

## BACKGROUND ART

An L-β-haloalanine, particularly L-β-chloroalanine, is an amino acid possessing a physiological activity per se (J. Biol. Chem., Vol. 252, pp. 3170), and a compound available as an intermediate for synthesizing medical products, pesticides, and the like, and accordingly, attempts have been made to develop processes for advantageously producing such an amino acid. These processes are mainly based on chemical syntheses (for example, see Japanese Examined Patent Publication (Kokoku) No. 58-22140 and Japanese Unexamined Patent Publication (Kokai) No. 57-188548).

The conventional synthesis processes, however, necessarily form the D,L-racemate, and moreover, since an optically active product is desirable as an intermediate for physiologically active substances, which is its main application, the conventional processes involve an optical resolution stage. Such an optical resolution means has also been developed (see Japanese Unexamined Patent Publication (Kokai) No. 61-152645), but cannot be necessarily considered to have a good total production efficiency.

Therefore, the object of the invention is to provide a process for efficiently producing an optically active haloamino acid, i.e., L-β-chloroalanine or L-β-bromoalanine.

The present inventors made an extensive search for an enzymatic process, to thereby solve the above-mentioned problem, and unexpectedly found that an enzymatic conversion system, which uses an enzymatically active substance to effect an amination with an asymmetric reduction of a 2-ketobutyric acid, from 2-ketobutyric acid to an L-amino acid (for example, see W. Hummel and M. R. Kula., Eur. J. Biochem. 184, 1-13 (1989)) can be applied to a corresponding 3-halo-2-ketobutyric acid, even though it has a considerable bulk substituent, to thereby achieve the present invention.

## DISCLOSURE OF INVENTION

In accordance with the present invention, there is provided a process for producing L-β-chloroalanine (or 2-amino-3-chlorobutyric acid) or L-β-bromoalanine (or 2-amino-3-bromobutyric acid), characterized by bringing 3-chloro-2-ketobutyric acid or 3-bromo-2-ketobutyric acid into contact with alanine dehydrogenase in the presence of a reduction type nicotinamide adenine dinucleotide (NADH) and an $NH_3$ source.

The process mentioned above, in which an NADH regeneration system coexists in the reaction system and permits this process to be carried out more economically, is also provided.

## BEST MODE OF CARRYING OUT THE INVENTION

The reduction type nicotinamide adenine dinucleotide (NADH) used in the invention is commercially available and can be bought from various suppliers. Alternatively, it can be synthesized by a process known per se, for example, by extraction from an yeast, or by condensation between nicotinamide mononucleotide and adenosine-5'-phosphate.

As $NH_3$ sources, inorganic ammonium salts such as ammonium hydroxide, ammonium chloride, ammonium nitrate, ammonium carbonate, and ammonium sulfate, and organic ammonium salts such as ammonium formate, ammonium acetate, ammonium citrate, and ammonium oxalate can be mentioned. Of these, in the case where the NADH regeneration system which utilizes formate dehydrogenase coexists in the reaction system, the use of ammonium formate, which also serves as a substrate for said enzyme, is particularly preferable.

3-chloro-2-ketobutyric acid and 3-bromo-2-ketobutyric acid which are a starting material or substrate in the reaction of the process, are known compounds per se, and a commercially available compound can be used as such. Of these, 3-chloro-2-ketobutyric acid is preferable. The enzymatically active substance used to effect an amination with asymmetric reduction in accordance with the present invention includes any active substance, independent of the origin and purity thereof, as long as it has an enzymatic activity that can convert said substrate to corresponding L-β-haloalanine in the presence of the NADH and $NH_3$ source. Such active substances specifically include an alanine dehydrogenase (EC 1,4,1,1), leucine dehydrogenase (EC 1,4,1,9), phenylalanine dehydrogenase (EC 1,4,1 - ) and a composition containing one of the same. Although the substances may be prepared from an animal, plant or microorganism origin, the substance

originated from a microorganism is most convenient for use in the invention.

Each of the enzymes can be widely prepared from various microorganisms. For example, microorganisms useful for a preparation of the alanine dehydrogenase include those belonging to the genus Bacillus such as Bacillus subtillis, Bacillus stearothermophilus; the genus streptomyces such as Streptomyces phaeochromogenes, Streptomyces hygroscopicus; the genus Micrococcus such as Micrococcus luteus; the other genus such as Flavobacterium gasogenes, Brevibacterium linens, and the like. The microorganisms useful for a preparation of the leucine dehydrogenase include those belonging to the genus Bacillus such as Bacillus sphaericus, Bacillus stearothermophilus; the genus Staphylococcus such as Staphylococcus aureus; and the genus Hafnia such as Hafnia alvei, and the like. The microorganisms useful for a preparation of the phenylalanine dehydrogenase include those belonging to the genus Bacillus such as Bacillus badius, Bacillus sphaericus; the genus Nocardia such as Nocardia opaca; the genus Rhodococcus such as Rhodococcus maris; the genus Sporasarcina such as Sporasarcina ureae; the genus Thermoactinomyces such as Thermoactinomyces intermedius, and the like.

The enzymatically active substances include a purified enzyme originated from the microorganisms, cells of the microorganisms as they are, and homogenates of these cells, as well as the cells or cell homogenates immobilized on a water-insoluble carrier while maintaining an activity thereof. In particular, for the alanine dehydrogenase and leucine dehydrogenase, purified enzymes originated from the microorganism belonging to genus Bacillus, which are commercially available and have well known characteristics, are preferably used.

The contact between the substrate, 3-chloro-2-ketobutyric acid or 3-bromo-2-ketobutyric acid, and the enzymatically active substance which can effect an amination with an asymmetric reduction of 2-ketobutyric acid is carried out in the presence of NADH and the $NH_3$ source. The term "contact" means that the substrate is subjected to the activity of the enzymatically active substance, and may be attained by standing, agitating, mixing, or any other means. Although the optimum amount of each component existing in this reaction system depends upon the type of enzyme to be used and the reaction manner, and should not be restricted, the alanine dehydrogenase is generally in the range of 1-100 U/mℓ, preferably 10-20 U/mℓ, expressed by its active unit, NADH is generally in the range of 0.001-0.1 M, preferably 0.002-0.01 M, and the substrate is generally in the range of 0.01-1 M, preferably 0.05-0.1 M.

Furthermore, the coexistence of the NADH regeneration system enables the present invention to produce an L-$\beta$-haloalanine more efficiently. The term "regeneration system" as used herein means a system in which $NAD^+$ (oxidization type nicotinamide adenine dinucleotide) converted from NADH associating with the enzymatic conversion of 3-chloro-2-ketobutyric acid or 3-bromo-2-ketobutyric acid to the corresponding L-$\beta$-haloalanine can be regenerated into NADH. Any system can be used as long as it does not adversely affect the above-described conversion. Systems which utilize microorganisms which produce hydrogen under a hydrogen atmosphere (for example, see Japanese Unexamined Patent Publication (Kokai) No. 61-67495), and systems which utilize the activity of formate dehydrogenase (EC 1.2.1.2.) can be mentioned as such systems. The latter has already been known to catalyze the following reaction:

$$HCOO + NAD^+ \rightleftharpoons CO_2 + NADH,$$

and any of these systems can be used in the present invention. Preferably, a system is used which possesses reaction conditions similar to those of the above-mentioned L-$\beta$-haloalanine formation system. Since such enzymes are widely distributed in the plants and microorganisms, an enzyme purified therefrom or a cell-treated product thereof may be used. More specifically, purified enzymes originated from various yeasts, e.g., Candida boidinii and Candida methanolica, bacterium, e.g., Escherichia coli, Paracoccus SP., Pseudomas oxalaticus, Clostridium termoeaceticum, and plants, e.g., pea (Pisum sativum L.) and wild soybean (Glycine soja Sieb. et Zucc.), and cytolysates thereof can be used. The preparation of these enzymes, etc., are known from literatures (for example, see European Journal of Biochemistry, 62, 151-(1976); FEMS Microbiology Letters, 48, 139(1987); Journal of Bacteriology, 170, 3189(1988); and Eur. J. Biochem., 83, 485(1978)). It is also essential that formate ions coexist in the system where the formate, dehydrogenase is used. As the source of formate ion, a free formic acid and various formates can be used, but ammonium formate, which can also be utilized as the above-mentioned $NH_3$ source, is preferably used. Although the amount of formate dehydrogenase in the reaction system should not be restricted, it is generally used within the range of 1-50 U/mℓ, and preferably used within the range of 5-20 U/mℓ, as expressed by the active unit, and ammonium formate is generally used within the range of 0.1-5 M, and preferably used within the range of 0.5-2 M.

In these reaction systems, the reaction may be carried out in a buffer solution which is usually used, at

a pH value of 6-9, particularly a pH value of 6-7 in the case of the production of L-β-chloroalanine, or a pH value of 6-6.5 in the case of the production of L-β-bromoalanine. The reaction temperature is usually set at 20-60°C, and preferably set at 20-30°C. Preferable buffers are potassium phosphate, Tris•HCl, MOPS, MES and HEPES.

The reaction may be carried out either batchwise or continuously. It is important that the reaction system is constructed so that the period of the contact between the substrate, 3-halo-2-ketobutyric acid and the enzymatically active substance that can be effected amination with asymmetric reduction of 2-ketobutyric acid, is as short as possible, from the view point of preventing the substrate from being decomposed into side-products.

The isolation and purification of the resulting reaction mixture of L-β-chloroalanine or L-β-bromoalanine can be carried out with ease by any of the various methods for purifying an amino acid known per se, for example, by using a cation-exchanging resin and/or an anion exchanging resin.

[Example]

The present invention will now be illustrated with reference to the following working examples.

Example 1 Production of L-β-Chloroalanine (using alanine dehydrogenase)

To 300 mM phosphate buffer (20 mℓ) containing ammonium 3-chloro-2-ketobutyrate (2 mmol), ammonium formate (10 mmol), and NAD (20 μmol) were added 400 units of alanine dehydrogenase originated from Bacillus stearothermophilus and 80 units of formate dehydrogenase originated from Candida boidinii. After incubation at 30°C for 1 hour, ammonium 3-chloro-2-ketobutyrate (2 mmol) and ammonium formate (2 mmol) were added thereto, and the mixture was incubated for another 1 hour. Furthermore, ammonium 3-chloro-2-ketobutyrate (2 mmol) and ammonium formate (2 mmol) were similarly added, and the mixture was incubated for 1 hour to yield 5.4 mmol (yield: 90.0%) of the desired L-β-chloroalanine in the reaction mixture.

The isolation of the product was carried out by purification with Dowex-50-W (trademark of Dow Chemical; $H^+$-form: cation-exchanging resin), and subsequently with Amberlite CS-400 (trademark of by Organo Co., Ltd., $Cl^-$ -form: anion-exchanging resin), whereby 0.7 g of pure product of L-β-chloroalanine was able to be yielded.

The product in the form of hydrochloride had an optical rotation of $[\alpha]_D^{20}$ = -3.8°, and confirmed by a high performance liquid chromatography using an optical resolution column (Crown Pack CR (-), produced by Daicel Chemical Industries) to be an L-isomer having an optical purity of almost 100% (Value in the literature: $[\alpha]_D^{20}$ = -4°).

Alanine Dehydrogenase

A commercially available enzyme (produced by Unitika Ltd.) was used as such, or cell homogenate supernatant of the latter logarithmic growth phase in which the above microorganisms had been cultured in a usual manner was used.

Formate Dehydrogenase

A commercially available enzyme (produced by Boehringer Mannheim-Yamanouchi, Production No.:837016) was used.

Examples 2 to 7 Production of L-β-Chloroalanine Using Cell-Free Extract (Homogenate) of Various Microorganisms as Alanine Dehydrogenase

The following strains (the existence of alanine dehydrogenase has been shown in literature, etc.) were cultured in a medium containing 0.3-0.5% L-alanine as enzyme-inducer in a manner similar to that of Example 1 to prepare cell-free extracts (homogenates). The activity of alanine dehydrogenase was determined in each case, and the synthesis of L-β-chloroalanine was carried out using the following composition.

| Composition | |
|---|---|
| 3-Chloro-2-ketobutyric Acid | 50 mM |
| Ammonium Formate | 400 mM |
| Phosphate Buffer | 400 mM |
| $NAD^+$ | 1 mM |
| Cell Homogenate (0.04-1.3 Units of alanine dehydrogenase) | |
| Formate Dehydrogenase | 4 Units |
| | Total 1 mℓ |

The yields of L-β-chloroalanine after incubation at 30°C for 6 hours are shown in the following Table 1.

Table 1

| Ex. No. | Strain | Activity of Alanine Dehydrogenase (U/mℓ homogenate) | Yield of β-Chloroalanine (%) |
|---|---|---|---|
| 2 | Streptomyces phaeochromogenes IFO 3149 | 2.7 | 14.7 |
| 3 | Streptomyces hygroscopicus ATCC 15484 | 3.5 | 32.5 |
| 4 | Bacillus sphaericus ATCC 102088 | 0.89 | 3.0 |
| 5 | Flavobacterium gasogenes IFO 12065 | 1.2 | 19.5 |
| 6 | Brevibacterium linens ATCC 8377 | 0.53 | 5.2 |
| 7 | Micrococcus luteus ATCC 9341 | 0.10 | 6.3 |

Example 8 Production of L-β-Bromoalanine (Using of alanine dehydrogenase)

The procedure of Example 1 was repeated, except that the following composition was incubated at 30°C for 10 minutes, to give a yield of 1.5% of L-β-bromoalanine.

| Composition | |
|---|---|
| 3-Bromo-2-ketobutyric Acid<br>Ammonium Formate<br>Phosphate Buffer<br>$NAD^+$<br>Alanine Dehydrogenase (Bacillus stearothermophilus origin)<br>Formate Dehydrogenase (Canada bolidini origin) | 50 mM<br>400 mM<br>400 mM<br>0.25 mM<br>13 Units<br>4 Units |
| | Total 1 mℓ |

Example 9 Production of L-β-Chloroalanine (Using of leucine dehydrogenase)

The produce of Example 1 was repeated, except that 500 units of leucine dehydrogenase originated from Bacillus stearothermophilus (produced by Unitika, LTD.) instead of the alanine dehydrogenase were used, and separately incubated for 30 minutes, to yield 1.7 mmol (yield: 28.0%) of the desired L-β-chloroalanine in the reaction mixture. The reaction mixture was treated by following the procedure of Example 1, to yield 0.2 g of purified L-β-chloroalanine.

Examples 10 to 12 Production of L-β-Chloroalanine Using Cell-Free Extract (Homogenate) of Various Microorganisms as Leucine or Phenylalanine Dehydrogenase Active Substance

As described in above Examples 2 to 7, the leucine or phenylalanine dehydrogenase active substance and the following composition were used to yield L-β-chloroalanine.

| Composition | |
|---|---|
| 3-Chloro-2-ketobutyric Acid<br>Ammonium Formate<br>Phosphate Buffer (pH 7.5) (note: Only Ex.12 was used Tris•HCl buffer (pH 8.5))<br>$NAD^+$<br>Cell Homogenate (0.001-4.0 units of leucine or phenylalanine dehydrogenase activity)<br>Formate Dehydrogenase | 100 mM<br>400 mM<br>400 mM<br>1 mM<br><br>4 Units |
| | Total 1 mℓ |

The yields of L-β-chloroalanine after incubation at 30 °C for 2 hours are shown in the following Tables 2 and 3.

Table 2

| Ex. No. | Strain | Activity of Phenylalanine Dehydrogenase (U/mℓ homogenate) | Yield of β-Chloroalanine (%) |
|---|---|---|---|
| 10 | Bacillus sphaericus IFO 3525 ATCC 10208 | 0.1 | 3.1 |
| 11 | Staphylococcus aureus IFO 3060 | 0.001 | 0.3 |

Table 3

| Ex. No. | Strain | Activity of Phenylalanine Dehydrogenase (U/mℓ homogenate) | Yield of β-Chloroalanine (%) |
|---|---|---|---|
| 12 | Bacillus badius IAM 11059 | 4.0 | 2.0 |
| 13 | Sporosarcina urea IFO 12699 ATCC 6473 | 0.06 | 6.0 |
| 14 | Thermoactinomyces intermedius IFO 14230 ATCC 33205 | 0.1 | 7.3 |

Example 15 Production of L-β-Bromoalanine (Using of leucine dehydrogenase)

The procedure of Example 1 was repeated, except that the following composition of 3-bromo-2-ketobutyric acid and other reagents were used and incubated at 30°C for 10 minutes, to give a yield of 1.6% of L-β-bromoalanine.

| Composition | |
|---|---|
| 3-Bromo-2-ketobutyric Acid | 50 mM |
| Ammonium Formate | 400 mM |
| Phosphate Buffer (pH 7.5) | 400 mM |
| $NAD^+$ | 1 mM |
| Leucine Dehydrogenase (Bacillus stearothermophilus origin) | 25 Units |
| Formate Dehydrogenase (Candida boidinii origin) | 4 Units |
| | Total 1 mℓ |

Example 16 Production of L-β-Bromoalanine (Using of phenylalanine dehydrogenase)

The procedure of Example 15 was repeated, except that 17 units of phenylalanine dehydrogenase originated form Bacillus badius instead of leucine dehydrogenase, to give L-β-bromoalanine at a yield of 3.7%.

Industrial Applicability

In accordance with the present invention, a process for producing an optically active L-haloamino acid is provided. Thus, the present invention is not only useful for a L-haloamino acid producer, but also contributes to technical developments in various industries in which the inventive products are utilized for producing a physiologically active compound as a intermediate, and an end product therefrom.

## Claims

**1.** A process for producing L-β-chloroalanine or L-β-bromoalanine, characterized by bringing a substrate, 3-chloro-2-ketobutyric acid or 3-bromo-2-ketobutyric acid, into contact with an active substrate which can be effected amination with asymmetric reduction of a 2-ketobutyric acid in the presence of a reduction type nicotinamide adenine dinucleotide (NADH) and an $NH_3$ source.

**2.** The process in accordance with claim 1, wherein the active substance which can aminated with an asymmetric reduction of a 2-ketobutyric acid is selected from the group consisting of an alanine dehydrogenase active substance, leucine dehydrogenase active substance and phenylalanine dehydrogenase active substance.

3. The process in accordance with claim 1, which comprises a regeneration system of NADH.

4. The process in accordance with claim 3, wherein the regeneration system of NADH comprises a formate dehydrogenase and ammonium formate.

5. The process in accordance with claim 1, wherein the substrate is 3-chloro-2-ketobutyric acid, and the active substance which can be aminated with an asymmetric reduction of 2-ketobutyric acid, is an alanine dehydrogenase active substance.

6. The process in accordance with claim 3, wherein the substrate is 3-chloro-2-ketobutyric acid, and the active substance which can be aminated with an asymmetric reduction of 2-ketobutyric acid, is an alanine dehydrogenase active substance.

# INTERNATIONAL SEARCH REPORT

International Application No PCT/JP91/01150

**I. CLASSIFICATION OF SUBJECT MATTER** (if several classification symbols apply, indicate all)[6]

According to International Patent Classification (IPC) or to both National Classification and IPC Int. $Cl^5$

C12P13/06//(C12P13/06, C12R1:07), (C12P13/06, C12R1:55), (C12P13/06, C12R1:465), (C12P13/06, C12R1:20),

**II. FIELDS SEARCHED**

Minimum Documentation Searched[7]

| Classification System | Classification Symbols |
|---|---|
| IPC | C12P13/06 |

Documentation Searched other than Minimum Documentation to the Extent that such Documents are Included in the Fields Searched[8]

Biological Abstracts Data Base (BIOSIS)

**III. DOCUMENTS CONSIDERED TO BE RELEVANT**[9]

| Category * | Citation of Document,[11] with indication, where appropriate, of the relevant passages[12] | Relevant to Claim No.[13] |
|---|---|---|
| Y | JP, A, 61-128895 (Takara Shuzo Co., Ltd.), June 16, 1986 (16. 06. 86), (Family: none) | 1-6 |
| Y | JP, A, 60-184393 (Ajinomoto Co., Inc.), September 19, 1985 (19. 09. 85), (Family: none) | 1-6 |

* Special categories of cited documents:[10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| November 18, 1991 (18. 11. 91) | December 9, 1991 (09. 12. 91) |
| **International Searching Authority** | **Signature of Authorized Officer** |
| Japanese Patent Office | |

(Information concerning IPC to be added in the column I)

(12P13/06, C12R1:13), (C12P13/06, C12R1:265), (C12P13/06, C12R1:445), (12P13/06, C12R1:01)